(19) Europäisches Patentamt

European Patent Office

Office européen des brevets

(11) Publication number: **0 511 499 A2**

## EUROPEAN PATENT APPLICATION

(21) Application number: **92105366.6**

(22) Date of filing: **27.03.92**

(51) Int. Cl.⁵: **A61M 25/00, A61B 5/00**

(30) Priority: **02.04.91 US 679377**

(43) Date of publication of application:
**04.11.92 Bulletin 92/45**

(84) Designated Contracting States:
**BE CH DE FR GB IT LI NL**

(71) Applicant: **ADVANCED CARDIOVASCULAR SYSTEMS, INC.**
**3200 Lakeside Drive**
**Santa Clara California 95052(US)**

(72) Inventor: **Gifford, Hanson S.**
**71 Morton Street**
**Palo Alto, California 94303(US)**

(74) Representative: **Baillie, Iain Cameron et al**
**Ladas & Parry Altheimer Eck 2**
**W-8000 München 2(DE)**

(54) Method and catheter for controlled intravascular drug delivery.

(57) A vascular perfusion catheter includes a catheter body having a macroporous matrix forming at least a portion of its distal end. Blood flow is measured *in situ* by a Doppler mechanism operably coupled to the catheter body. A solution carrying a desired therapeutic agent may be introduced through the catheter and released through the macroporous matrix until a preselected blood flow is observed.

EP 0 511 499 A2

## BACKGROUND OF THE INVENTION

### 1. Field of the Invention

The present invention relates generally to apparatus and methods for intravascular drug delivery and more particularly to a catheter and method for the controlled infusion of therapeutic agents into an extended region within a blood vessel over a prolonged time period.

Catheter infusion of drugs and other active substances may be useful for treating a wide variety of disorders. Of particular interest to the present invention, catheters may be used for the localized administration of thrombolytic agents to dissolve clots within the vascular system. Typically, the catheter is percutaneously introduced to the vascular system with the distal end located adjacent to or within the region of clot or thrombus. The thrombolytic agent is then delivered through the catheter and released through one or more discrete perfusion ports formed near the distal end of the catheter. Alternatively, two or more catheters may be utilized simultaneously in an attempt to release the thrombolytic agent throughout the entire clotted region.

Although generally effective, such catheter designs suffer from certain disadvantages. In particular, during infusion of a thrombolytic agent, there is no accurate method for determining *in situ* the progress of thrombolysis. As a result, indirect measuring techniques must be employed, for example, external blood pressure measurement, external Doppler devices, and, in the case of coronary arteries, monitoring electrical signals from the heart. Each of these alternatives requires additional equipment and effort, while providing at best a rough measurement of the progress of thrombolysis.

Direct or *in situ* measurement techniques exist per se, but these entail additional instrumentation and loss of time. After a course of thrombolysis, for example, the patient may be taken to an x-ray suite where an arteriogram is performed to monitor the progress of thrombolysis. While this technique provides the accuracy of direct measurement, it is particularly time consuming and poses additional risk for patients who may be sensitive to the contrast media which are employed. Since eventual patient outcome is related to ischemia time, any increase in treatment time is highly undesirable.

A particular disadvantage with prior direct techniques is the inability to provide information on a real-time basis, i.e., *in situ* measurements during the course of the treatment. Even discounting the loss of time and additional risks to a patient, the information provided by prior techniques is at best a static measurement at a single period of time. By the time the information is available or interpreted, the patient's status may be substantially changed.

Since immediate feedback has not been available to physicians, thrombolysis treatment has been conducted in a highly qualitative manner. Consequently, patients may receive grossly excessive or inadequate amounts of thrombolytic agents. In the former case, drug side effects are increased. In the latter case, treatment is prolonged with increased risk of catheterization complications.

Thus, it is desirable to provide improved apparatus, catheters, and methods for the localized intravascular delivery of therapeutic agents, e.g., clot dissolving agents, for a period of time which is directly related to the resolution of thrombotic and stenotic lesions. The catheter should be suitable for percutaneous introduction to a desired location within the vascular system, preferably utilizing conventional guide wire introduction techniques. The catheter should further be able to provide controllable delivery rates within a vessel and means for monitoring blood flow within the vessel. Thus, the blood flow may be conveniently determined at all times. It would be further desirable if the catheters were suitable for vascular placement over extended periods of time.

### 2. Description of the Background Art

U.S. Patent No. 4,765,339 describes a catheter having a tubular dialysis membrane formed thereon. The catheter is intended primarily for blood analysis, but it is suggested that it would also be useful for introducing medicaments. It would not, however, be useful for introducing macromolecules as the membrane is microporous and selected to block proteins and other larger molecules. U.S. Patent No. 4,671,287 describes a catheter having an oxygen permeable bag intended for gastrointestinal oxygenation. U.S. Patent No. 4,274,417 describes a blood gas analysis probe having a permeable tip. U.S. Patent No. 4,318,402 describes a liquid infusion catheter having a perforated outer tube and an unperforated inner tube. U.S. Patent No. 4,717,379 describes a catheter probe having a plurality of radial capillary openings intended for the release of lubricants, washing agents, etc. U.S. Patent No. 4,068,664 describes a surgical suction wand having a perforated tip. U.S. Patent No. 3,528,427 describes a drainage cannula having a perforated tip and an inner tube. U.S. Patent No. 3,593,713 describes an infusion catheter with a perforated (foraminous) rigid shaft. United States Catheter, Inc. has developed a dilatation balloon catheter where the balloon has a plurality of laser-drilled holes which allow release of an inflating medium.

U.S. Patent No. 4,175,566 describes a catheter having a fluid-velocity flow probe at its distal end. U.S. Patent No. 4,771,788 describes a fluid velocity measuring guide wire adapted for placement in the coronary arterial tree. U.S. Patent No. 4,665,925 describes a steerable catheter having a Doppler crystal at its tip to measure the velocity of blood *in vivo*. U.S. Patent No. 4,733,669 describes a catheter having a mechanism for positioning a Doppler shift transducer against the side wall of a blood vessel. U.S. Patent No. 4,850,358 describes a method for inserting a plurality of sensors, including a pressure sensor, into a biological fluid vessel. U.S. Patent No. 4,901,731 describes a catheter having a pressure transducer for measuring pressure differentials between spaced locations in a vessel. U.S. Patent No. 4,920,967 describes a fluid velocity measuring guide wire adapted for placement in the coronary arterial tree. The disclosures of each of the foregoing references are hereby incorporated by reference.

## SUMMARY OF THE INVENTION

The present invention comprises an apparatus and method for the controlled intravascular introduction of therapeutic agents, particularly high molecular weight macromolecular therapeutic agents such as thrombolytic proteins and other polypeptides. The apparatus comprises a catheter having an elongate catheter body with proximal and distal ends. At least one lumen extends axially from the proximal end of the catheter body to a drug delivery means which is located on the catheter body, typically being at or near the distal end of the catheter body. The apparatus further comprises a means for measuring blood flow past the catheter body. Additionally, the apparatus includes a means for activating an alarm when the measured blood flow exceeds a preselected value.

The drug delivery means can comprise discrete perfusion ports, diffusion membranes, perforated tips or outer tubes, or the like. In a preferred embodiment, the drug delivery means is a macroporous matrix with permeability characteristics which permit the controlled infusion (under a pressure gradient) of macromolecules from the lumen to a region within a blood vessel which surrounds the matrix. In the embodiment, the macroporous matrix has a tubular geometry and forms a portion of the exterior of the catheter body, extending over a desired axial length. In this way, highly uniform and controlled flow of the therapeutic agent can be achieved over extended lengths of the catheter body in a manner which is unattainable with the perforate structures heretofore employed.

The means for measuring blood flow may be one of many types, including pressure transducers, electromagnetic detectors, ultrasonic detectors, thermodilution flow measurement means, or any combination thereof. In a preferred embodiment, Doppler ultrasound flow measurement means are provided by mounting one or more Doppler crystals along or within the catheter body.

The number and position of crystals may be varied. In one embodiment, one or more crystals are mounted proximal to the drug delivery means. In another embodiment, a pair of crystals is mounted distal to the drug delivery means. In a preferred embodiment, two or more Doppler crystals are mounted on opposing sides of the catheter distal to the matrix and are positioned to face distally. Alternative embodiments may be constructed by varying the position and number of Doppler crystals employed without departing from the principles of the present invention.

In the method of the present invention, a perfusion catheter is percutaneously introduced and transluminally positioned so that the drug delivery means is located within or adjacent to a region within a blood vessel which requires therapy, usually a region of stenosis and/or thrombus. A desired therapeutic agent may then be introduced through the lumen, such as a solution containing a thrombolytic polypeptide. Concurrently, blood flow within the blood vessel is determined *in situ* by measuring means. The delivery rate of the therapeutic agent can be carefully controlled in response to the blood flow observed.

## BRIEF DESCRIPTION OF THE DRAWINGS

Fig. 1 is an isometric view of a controlled perfusion catheter constructed in accordance with the principles of the present invention.

Fig. 2 is a detailed elevational view of the distal end of the catheter of Fig. 1 shown in cross-section.

Fig. 3 is a cross-sectional view taken along line 3-3 of Fig. 2.

Fig. 4 is a cross-sectional view taken along 4-4 of Fig. 2.

Fig. 5 is a cross-sectional view taken along line 5-5 of Fig. 2.

Fig. 6 illustrates a first alternate embodiment of the catheter of the present invention.

Fig. 7 is a cross-sectional view taken along line 7-7 of Fig. 6.

Fig. 8 is a cross-sectional view taken along 8-8 of Fig. 6.

Fig. 9 is a cross-sectional view taken along line 9-9 of Fig. 6.

Fig. 10 illustrates a second alternate embodiment of the catheter of the present invention.

Fig. 11 is a cross-sectional view taken along line 11-11 of Fig. 10.

Fig. 12 is a cross-sectional view taken along 12-12 of Fig. 10.

Fig. 13 is a cross-sectional view taken along line 13-13 of Fig. 10.

Fig. 14 illustrates a third alternate embodiment of the catheter of the present invention.

Fig. 15 is a cross-sectional view taken along line 15-15 of Fig. 14.

Fig. 16 is a cross-sectional view taken along 16-16 of Fig. 14.

Fig. 17 is a cross-sectional view taken along line 17-17 of Fig. 14.

Fig. 18 illustrates the method of the present invention which utilizes a perfusion catheter and a flow monitoring means to introduce a thrombolytic polypeptide to a region of thrombus within a patient's vascular system.

## DESCRIPTION OF THE PREFERRED EMBODIMENTS

The present invention is useful for intravascular delivery of a wide variety of therapeutic agents for treating vascular lesions, e.g., tissue plasminogen activator (TPA), streptokinase, urokinase, heparin, vasodilators, and the like. The invention is particularly useful for site-specific delivery of those agents which may influence blood flow within a particular vessel.

The following discussion will focus on the treatment of thrombus and plaque within the vascular system using thromholytic polypeptides which typically increase blood flow, such as TPA. The present invention, however, is not limited to such treatment and instead encompasses the delivery of other macromolecules and smaller drugs which may be advantageously released into the vascular system while monitoring blood flow using the catheter and method of the present invention.

The catheter of the present invention comprises an elongate, flexible catheter body having proximal and distal ends. The length and diameter of the catheter body will vary depending on the intended application, typically having a length in the range from about 60 to 150 cm and a diameter in the range from about 3 to 11 F (one French (F) is equal to 0.33 mm). When the catheter is intended to reach the coronary blood vessels, the catheter body will typically have a length in the range from about 120 to 150 cm and a diameter in the range from about 3 to 8 F. When intended to reach the peripheral blood vessels, the catheter body will usually have a length from about 60 to 150 cm and a diameter from about 3 to 11 F.

The catheter body may include one or more tubular elements with multiple tubes usually being arranged coaxially. The tube(s) will typically be formed by extrusion of an organic polymer, typically a thermoplastic such as nylon, polyurethane, polyethyleneterephthalate (PET), polyvinylchloride (PVC), polyethylene, or the like. The tubes so formed may be reinforced or unreinforced, with reinforcement being optionally provided by metal wires, metal braided cables, or the like. Processes and techniques for forming intravascular catheter bodies are well known in the art and well described in the patent, scientific, and medical literature.

The tube(s) will define one or more lumens extending axially within the catheter body from the proximal end. At least one lumen will be provided for delivering the therapeutic agent from the proximal end of the catheter to near the distal end, as described in more detail hereinafter. Additional lumens may be provided for a variety of purposes, such as to allow introduction and placement of the catheter over a guide wire, and the like. Alternatively, the catheter may employ a fixed guide wire at its distal end to allow for positioning of the catheter within the vascular system.

In a particular embodiment, one or more additional lumens are provided to deliver different therapeutic or other agents to different locations on the catheter. For example, while a thrombolytic agent is being delivered to the distal end of the catheter, a separate lumen might simultaneously deliver an anti-clotting agent along the remaining length of the catheter to inhibit clotting which might otherwise be induced by the catheter.

The catheter of the present invention will include or be attachable to a proximal housing which provides for access to the internal lumen(s) within the catheter body. The proximal housing will include one or more conventional fittings, e.g., luer connectors, which provide for attachment of tubes and introduction of guide wires, as described in more detail hereinafter.

The therapeutic agent delivered by the catheter will be released under a preselected pressure gradient through a drug delivery means disposed along an axial length of the catheter body, usually although not necessarily disposed near the distal end of the catheter. The drug delivery means will be in fluid connection with the lumen which carries the therapeutic agent so that the matrix provides a rate-controlling barrier for releasing the therapeutic agent from the catheter.

It will be appreciated by those skilled in the art that many structural alternatives can serve as the drug delivery means. For example, in U.S. Patent 4,318,402 issued to Vaillancourt, a multi-lumen infusion catheter with a perforated outer tube is disclosed which may serve as an appropriate delivery means. Alternatively, the delivery means may comprise a plurality of radial capillary openings as described in U.S. Patent No. 4,717,379, or a perforated tip as described in U.S. Patent Nos. 4,068,664 and 3,528,427. Other alternative designs include an infusion catheter with a perforated (foraminous) rigid shaft described in U.S. Patent No. 3,593,713, or a dilatation balloon catheter where the balloon has a plurality of laser-drilled holes which allow release of an inflating medium as developed by United States Catheter, Inc. The disclosures of each of the foregoing references are hereby incorporated by reference. Still other drug delivery means exist. For example, a catheter having multiple lumens, each with a discrete distal sidehole, is available from different vendors including Advanced Cardiovascular Systems of Mountain View, CA, as model EDM, and from Mallinkrodt of St. Louis, MO.

In a preferred embodiment, the drug delivery means is a macroporous matrix that forms a portion of the outer surface of the catheter body with the lumen disposed along the entire interior length of the matrix. In this way, the therapeutic agent can perfuse outward through the macroporous matrix to the exterior environment surrounding the catheter in a highly uniform and controlled manner, where the rate of perfusion is controlled both by the characteristics of the matrix and by the level of internal pressurization as described in more detail hereinafter. It would be possible, of course, to provide additional structure on the catheter to control or direct the flow of therapeutic agent in some manner.

In an exemplary embodiment, the macroporous matrix is formed as a close-ended tube or cylinder where the lumen opens into an interior volume within the tube. Such a structure is particularly advantageous since it provides for highly uniform diffusion in all radial directions along the entire length of the macroporous membrane. The length of the macroporous membrane may vary widely, usually being at least 1 cm in length and extending up to the entire catheter length, e.g., 160 cm. Usually, the length of the macroporous matrix will be in the range from about 2 to 50 cm, more usually being in the range from about 2 to 20 cm, and frequently being in the range from 5 to 15 cm.

Alternatively, the macroporous matrix may be formed as a sheath over an internal tubular structure which defines one or more therapeutic agent delivery lumens. Such a structure allows the sheath to be divided into two or more delivery zones where different agents may be simultaneously released from different locations on the catheter. The delivery zones may be axially spaced-apart, radially spaced-apart, or spaced-apart both axially and radially. In a particular embodiment, a first lumen can deliver a therapeutic agent to a macroporous matrix located near the catheter tip while a second lumen delivers an anti-clotting agent, such as heparin, to a macroporous matrix which extends over a portion of or the entire length of the catheter shaft. The ability to inhibit clot formation along the length of the catheter is particularly advantageous when the catheter is to be left in place for extended periods of time.

The construction of a catheter having a drug delivery means comprising a macroporous matrix is disclosed in co-pending application Serial No. 07/545,311, assigned to the assignee of the present application, the disclosure of which is hereby incorporated by reference.

The catheter of the present invention further includes one or more detection or measuring means disposed along or within the catheter body for monitoring blood flow and velocity concurrent with the delivery of therapeutic agent. In the embodiments adapted for the treatment of stenotic lesions and/or thrombosis, the measuring means is a pressure transducer, electromagnetic detectors, ultrasonic detectors, thermo-dilution flow measurement means, or the like. The use of pressure transducers in catheters is disclosed in U.S. Patent Nos. 4,850,358 and 4,901,731 issued to Millar, the disclosures of which are hereby incorporated by reference. Electromagnetic means for measuring fluid velocity in a catheter are disclosed in U.S. Patent No. 4,175,566 issued to Millar, the disclosure of which is hereby incorporated by reference. The use of Doppler means in catheters and guide wires is disclosed in U.S. Patent Nos. 4,665,925 and 4,771,788 issued to Millar, U.S. Patent No. 4,733,669 issued to Segal, and U.S. Patent No. 4,920,967 issued to Cottonaro et al., the disclosures of which are hereby incorporated by reference.

In a preferred embodiment, the measuring means comprises one or more Doppler crystals located along or within the catheter body for the detection of blood flow. Doppler crystals are known in the art. U.S. Patent No. 4,665,925, for example, teaches their general operation. The Doppler crystal produces acoustic tone bursts which are transmitted through the blood and reflected by various structures, including red blood cells, vessel walls, plaque, etc. Depending on the distance of each reflecting structure, the returning acoustic signals received by the Doppler crystal are separated in time.

In an alternative embodiment, the measuring means comprises one or more ultrasound crystals fabricated from an extruded tubular section of polymer, such as polyvinylidene (PVDF), so that the acoustic signal is transmitted axially from the tube edge. PVDF is available from Pennwalt Corp. under the tradename of Kynar. In this embodiment, the crystals are mounted in a distal end of a catheter having a guide wire therethrough. This construction permits the measurement of blood flow past the catheter while still accommodating the guide wire.

An adjustable receiver is incorporated to select signals reflected from the structures at a specified distance from the crystal. The reflected signals received by the Doppler crystal are amplified and compared in phase and frequency to the master oscillation signal of 20 megahertz, typically. The difference in frequency between the amplified reflected signal and the master or oscillator signal is the Doppler shift. The Doppler shift ($\delta f$) is defined by the Doppler equation:

$$\Delta f = 2F\ V/c\ COS\ \theta,$$

where F is the transmitted frequency, where V is the velocity of the fluid, where c is the velocity of sound in the fluid, and where $\theta$ is the angle between the fluid flow axis and the acoustic axis. The Doppler shift ($\Delta f$) is linearly related to the velocity of the fluid (V).

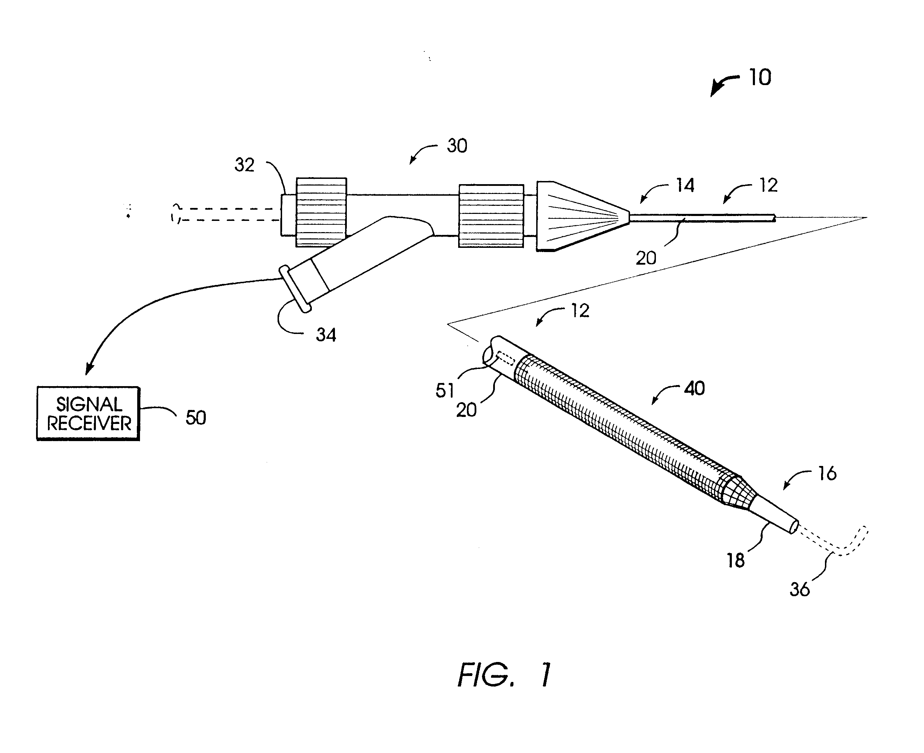

Referring now to Figs. 1-5, the construction of a first exemplary catheter 10 constructed in accordance with the principles of the present invention will be described. The catheter 10 comprises a catheter body 12 having a proximal end 14 and a distal end 16. The catheter body 12 includes an inner flexible tubular member 18 and an outer flexible tubular member 20. The inner flexible tube 18 has a central lumen 22 extending from proximal end 14 to distal end 16, while the inner tube 18 and outer tube 20 together define an annular lumen 24 which also extends from the proximal end to the distal end of the catheter.

A proximal housing 30 is secured to the proximal end 14 of catheter body 12. The housing 30 includes a central port 32 which communicates with the central lumen 22 of the inner flexible tube 18. The housing further includes a side port 34 which communicates with the annular lumen 24. Typically, the central lumen 22 and port 32 will be used to introduce the catheter 10 over a movable guide wire 36 (shown in broken line) in a conventional manner. The side port 34 will be used to introduce a solution carrying the therapeutic agent of interest.

Catheter 10 includes a tubular macroporous matrix 40 formed near the distal end 16. The distal end of tubular macroporous matrix 40 is attached to the outer surface of tubular member 18 in order to close the end of the annular lumen 24. The tubular macroporous matrix forms a continuous surface with the outer flexible tubular member 20 so that, in effect, the catheter body includes a single continuous outer tubular member having a non-porous or impermeable portion and a second porous portion defined by the tubular matrix 40. In this way, the solution carrying the therapeutic agent may be introduced through port 34, travel through the annular lumen 24, and be released under controlled conditions through the macroporous matrix 40.

The length of the tubular macroporous matrix 40 can vary widely within the limits set forth above. The porosity characteristics of the matrix 40 will generally be uniform over the entire surface area so that the release rate of the therapeutic solution will be the same at all locations. It would, of course, be possible to modify the porosity and other characteristics of the matrix 40 in cases where it is desired to provide a nonuniform release rate of the therapeutic solution.

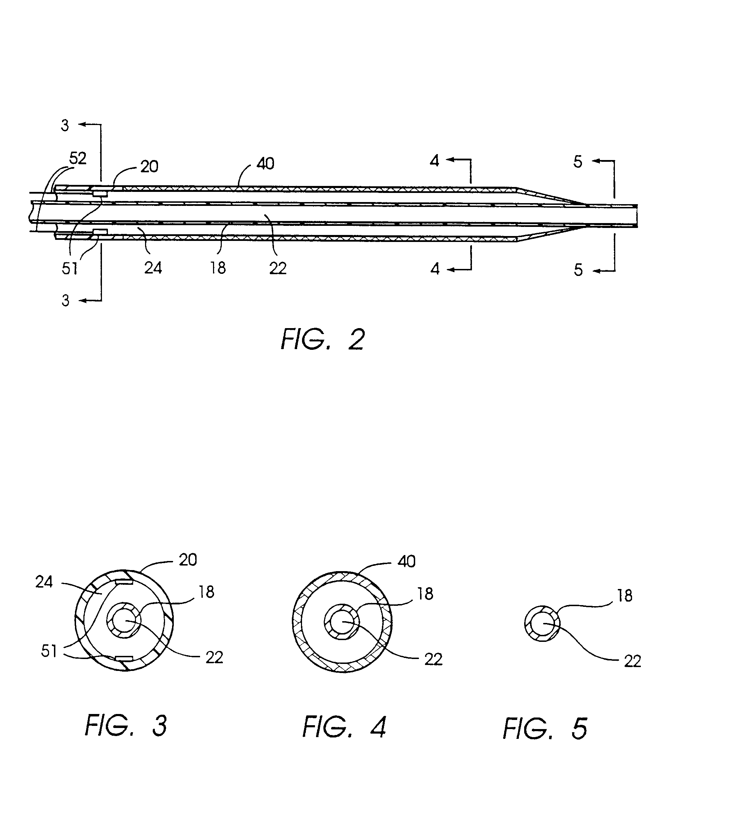

Catheter 10 further includes a Doppler mechanism which incorporates one or more Doppler crystals 51 connected to signal receiver 50 through electrical leads 52. As shown in the embodiment of Fig. 2, crystals 51 are secured to or within the inner wall of tubular member 20 proximal to matrix 40. Crystals 51, however, may be disposed in any manner which affords acoustic access to the blood vessel. For example, crystals 51 may be disposed within lumen 24 by conventional means, or secured to or within tubular member 18. In addition, crystals 51 may be provided with an acoustical backing (not shown) as is known in the art.

Crystals 51 are operably coupled to signal receiver 50 by electrical leads 52. Possible configurations for leads 52 abound. For example, leads 52 may be secured to tubular member 20 and/or tubular member 18. Alternatively, leads 52 may freely disposed within the lumen 24 (as shown). For convenience, leads 52 may be housed within a single coaxial or collinear cable.

In the embodiment of Figs. 1-5, any additional catheter diameter required to accommodate crystals extends only to the proximal end of the drug delivery means. Thus, the drug delivery means may be positioned proximate a lesion without any added bulk from the flow detection means. In treating a thrombotic lesion, for example, the drug delivery means and the distal tip of the catheter may be constructed of a smaller diameter which readily crosses narrow arteries and penetrates the thrombus.

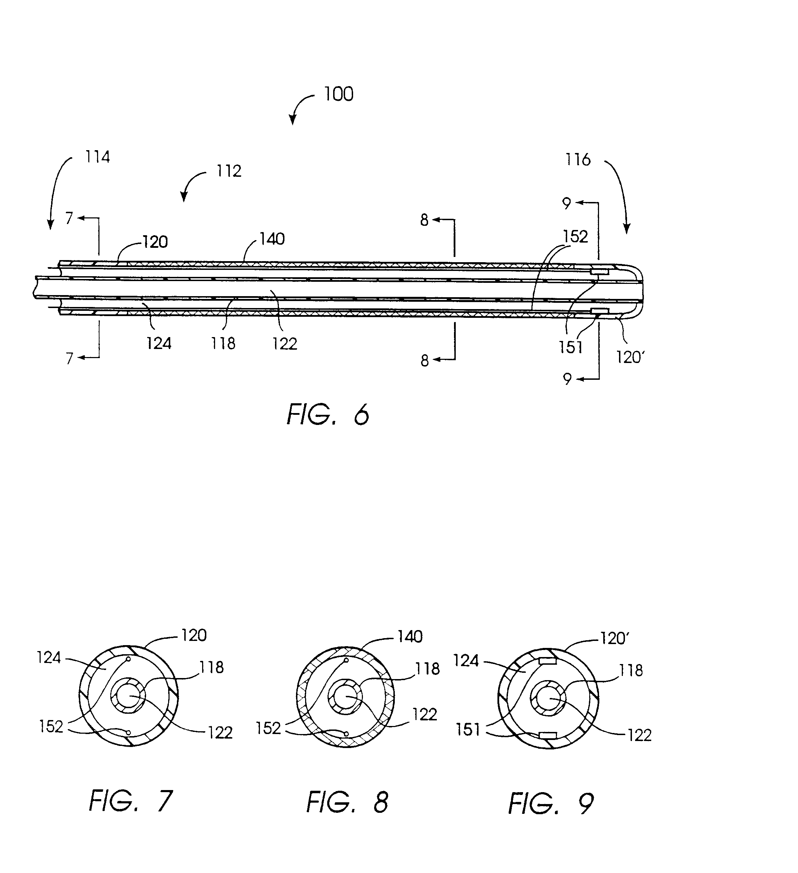

Alternative arrangements for the Doppler crystals exist. In the embodiment of Figs. 6-9, a catheter 100 includes a tubular macroporous matrix 140 formed near the atraumatic distal end 116 of catheter body 120. An inner flexible tube 118 has a central lumen 122 extending from proximal end 114 to distal end 116, while the inner tube 118 and outer tube 120 together define an annular lumen 124 which also extends from the proximal end to the distal end of the catheter.

As illustrated, Doppler crystals 151 are secured to tubular member 120' which is a portion of tubular member 120 just distal to the matrix 140. Other configurations exist. For example, crystals 151 may be disposed within lumen 124 by conventional means or secured to tubular member 118, with or without an acoustical backing.

Crystals 151 are operably coupled to receiver 50 through leads 152 which pass within lumen 124. Alternatively, leads 152 may be secured to tubular members 120', 140, 120, and/or 118, or any combination thereof. In catheter 100, however, leads 152 must extend a distance sufficiently distal to matrix 140 to reach crystals 151.

In the embodiment of Fig. 6, the acoustic axes of crystals 151 can be axially or radially aligned with catheter body 112, or a combination thereof. Since Doppler shift is usually more accurately determined when the crystals (acoustic axis) are aligned with fluid flow axis, an axial alignment gives improved signal stability and accuracy.

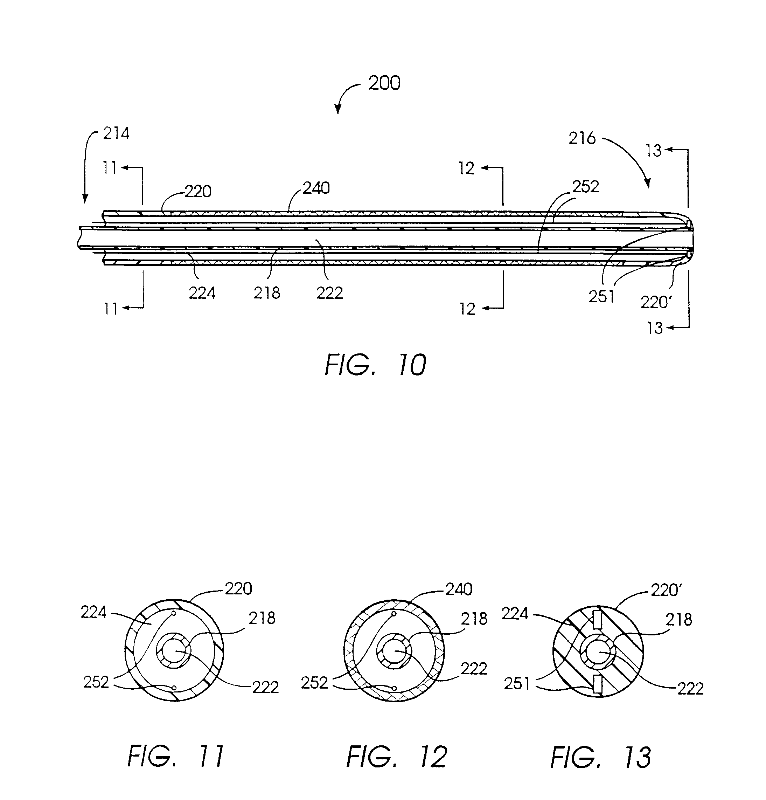

As yet another embodiment, there is shown in Figs. 10-13 a catheter 200 having a tubular macroporous matrix 240 formed near the atraumatic distal end 216 of catheter body 220. An inner flexible tube 218 has a central lumen 222 extending from proximal end 214 to distal end 216, while the inner tube 218 and outer tube 220 together define an annular lumen 224 which also extends from the proximal end to the distal end of the catheter.

As shown, Doppler crystals 251 are attached flushed at the distal end 216. While this embodiment requires crystals 251 to be located distally, the crystals 251 may be secured to or within the tubular members 218, 220, and/or lumen 252, or any combination thereof. The crystals may be further aligned axially or radially, or a combination thereof.

The embodiment of Fig. 10 has several advantages. The flush orientation of this embodiment positions the crystals away from any opposing vessel wall for greater crystal sensitivity. Furthermore, radially-oriented crystals positioned at the distal end of the catheter are better able to determine flow through a thrombosed segment of a vessel. In particular, the crystals can be positioned within or distal to the thrombosed segment with no acoustic interference from any proximal side branches of the vessel.

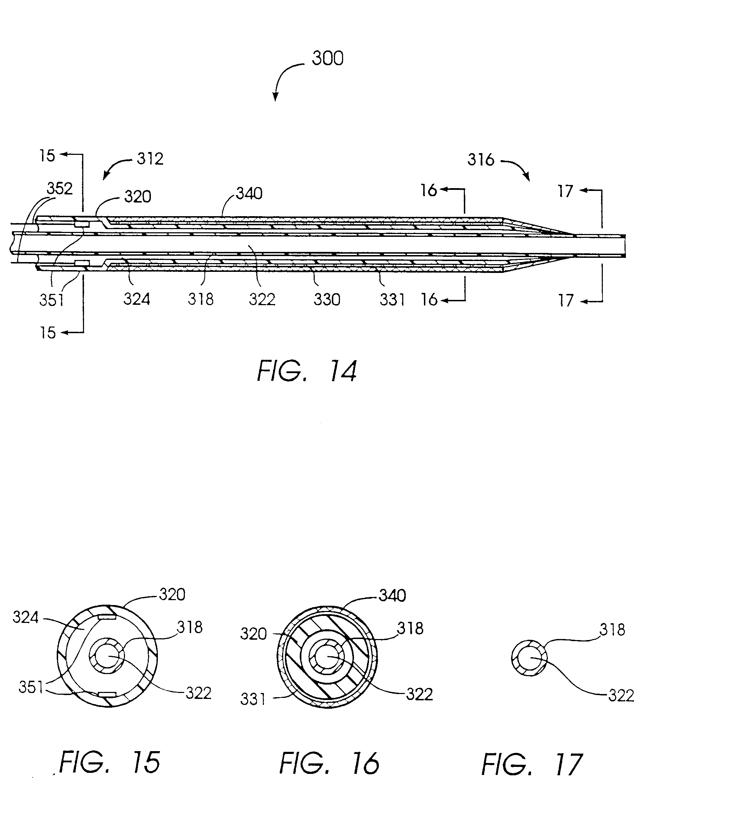

As yet another embodiment, in Figs. 14-17 there is shown a catheter 300 having an inner flexible tubular member 318 and an outer flexible tubular member 320. The inner flexible tube 318 has a central lumen 322 extending therethrough, while the inner tube 318 and outer tube 320 together define an annular lumen 324 with detection means 351, 352 disposed at a proximal end 312.

A tubular macroporous matrix 340 is secured about tubular member 320 near the distal end 316 of catheter body 320. An annular lumen 331 is defined between tubular member 320 and matrix 340. Tubular member 320 is provided with passageways (not shown), such as a plurality of laser-drilled holes, for the delivery of drug into lumen 331. A gold ribbon marker/spacer 330 is provided within the annular lumen 331 to further facilitate the delivery as well as provide a marker for radiographic identification. While not illustrated, it is understood that the embodiments of Figs. 1-13 may be similarly adapted to include the drug delivery means of Figs. 14-17.

Those skilled in the art will appreciate that many structural alternatives exist to the embodiments illustrated in the drawings. For example, the monitoring means can be installed in different orientations, including oblique orientation. This arrangement, however, may provide less accurate measurements of blood flow. There is no requirement that the monitoring means be placed in a symmetrical fashion. For example, it may be advantageous to place a first monitoring means at a location proximal to the delivery means and a second monitoring means at a location distal to the delivery means. Moreover, the number of Doppler crystals employed may be varied. Since it is not necessary to measure exact flow rates, a single Doppler crystal may be employed to determine whether or not flow has been restored. Furthermore, it will also be appreciated that the electrical leads can be routed through other passages out of possible contact with fluids.

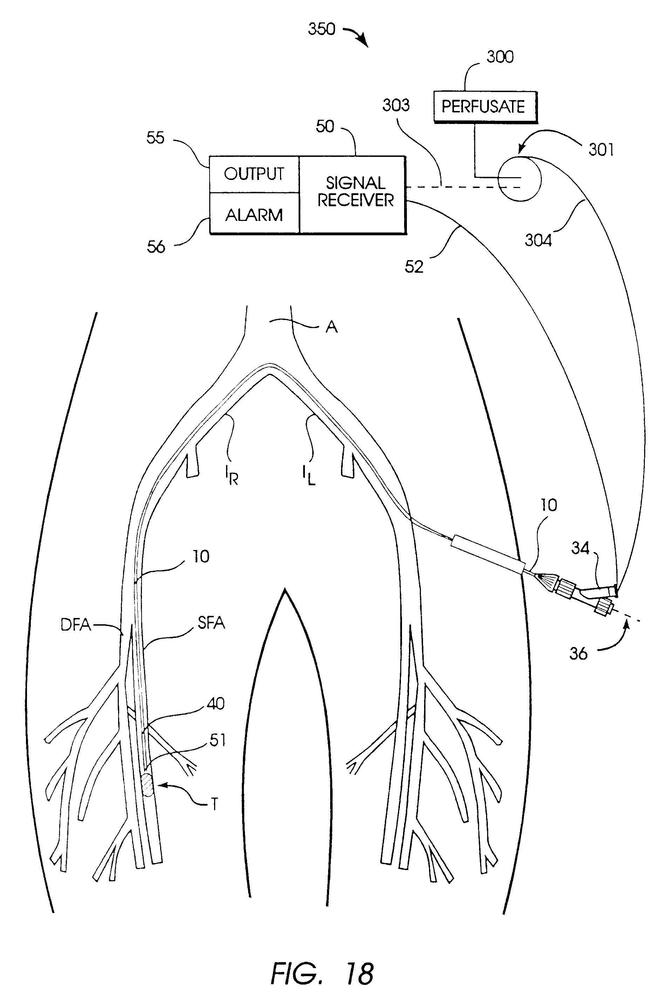

Referring now to Fig. 18, treatment of a region of thrombus **T** in a patient's superficial femoral artery **SFA** using the apparatus and method of the present invention will be described. The guide wire 36 is introduced through the left iliac artery $I_L$ into the right iliac artery $I_R$ and then into the superficial femoral artery **SFA** using an introducer catheter in a conventional manner. The guide wire 36 is positioned so that its distal end passes into the deep femoral artery **DFA** to reach the region of thrombus **T**. All such positioning steps can be performed under fluoroscopic guidance.

After the guide wire 36 has been properly positioned, the catheter 10 may be introduced by passage over the guide wire until the drug delivery means, such as macroporous membrane 40, lies within the region of thrombus **T**.

By positioning measuring means within the vessel, blood flow may be continually monitored *in situ*. In a preferred method, the measuring means, such as one or more Doppler crystals 51, is positioned within the vessel. The measuring means, however, may be introduced into the vessel independent of delivery means. In a less preferred method of the present invention, a separate catheter or guide wire means is employed for measuring blood flow while infusing drug. For example, drug is infused by a vascular catheter while blood flow past the catheter is measured by a Doppler tip wire guide which is introduced independently of the catheter. The construction of a Doppler tip wire guide is disclosed in U.S. Patent No. 4,771,788.

A perfusate solution containing the therapeutic agent of interest, typically a thrombolytic polypeptide, is then introduced through port 34 at an initial rate to at least partially dissolve the thrombus **T**. The perfusate is typically delivered from a reservoir 300, such as a flexible pouch, through a pump 301 which is connected to inlet port 34 by tubing 304. The pump will typically be capable of delivering a preselected volumetric flow rate over a wide range of pressures. Treatment conditions for two of the most commonly employed thrombolytic agents are as follows.

| Thrombolytic Agent | Volumetric Delivery Rate | Concentration Preferred Concentration |
|---|---|---|
| TPA | 5-150 cc/hr | 60 IU/cc to $10^6$ IU/cc; 250,000 to $10^6$ IU/cc |
| Urokinase | 5-150 cc/hr; 5-50 cc/hr | 3,000 IU/cc to 15,000 IU/cc; 1,000 IU/cc to 20,000 IU/cc |

The pressure of the perfusate in the catheter will be determined primarily by the flow rate applied by pump 301 and the resistance to flow provided by the macroporous membrane 40, and may vary from about 0.1 psi to 500 psi, usually being in the range from about 1 psi to 25 psi. The back pressure on the macroporous membrane 40 provides for highly uniform and controlled release of the thrombolytic agent throughout the region of thrombus **T** so that dissolution occurs at a constant rate, minimizing the total amount of agent required and reducing the chance that portions of the thrombus will be broken off and released as emboli.

Throughout the infusion procedure, the blood flow in the vessel is monitored by a Doppler system 350, which comprises crystals 51, leads 52, and receiver 50. The blood flow is determined by acoustic signals produced by crystals 51 at set intervals (or continuously). In response to acoustic reflections from the blood, signals are sent to receiver 50 through leads 52.

The Doppler system may further activate an output device 55, e.g., liquid crystal display and/or audio speaker, for indicating relative or absolute flow values. Additionally, an alarm 56 may be activated for signalling an operator when blood flow reaches or exceeds a target level. For the infusion of drugs which may decrease blood flow, alarm 56 may be activated when blood flow falls below a preselected level. For convenience, device 55 and alarm 56 may be housed within receiver 50.

In an alternative method, the delivery of the therapeutic agent is automatically titrated according to the measured blood flow, as determined by receiver 50. In this approach, pump 301, which is a variable-rate infusion pump under microprocessor control, is selectively coupled to receiver 50 via line 303, e.g., a serial communication line. Pump 301 may be programmed to deliver pre-determined rates in response to signals from receiver 50. In the simplest application of this technique, pump 301 would shut off after a target blood flow had been reached. In more advanced applications, pump 301 could be programmed to deliver a ramped flow rate (e.g., linear, exponentially declining, and the like) in response to changing blood flow. This method achieves maximum therapeutic response at an *in vivo* location while minimizing drug dosage and possible systemic side effects.

Although the foregoing invention has been described in detail for purposes of clarity of understanding, it will be obvious that certain modifications may be practiced within the scope of the appended claims.

## Claims

**1.** A vascular perfusion catheter comprising:

an inner flexible tubular member having a proximal end, a distal end, and a central lumen extending between the proximal and distal ends; and

an outer flexible tubular member disposed coaxially about the inner flexible tubular member and sealed thereto near the distal end to define an annular lumen having a closed distal end, wherein at least a portion of the outer flexible tubular member near the distal end is formed from a macroporous matrix material which will allow the controlled diffusion of macromolecules from the annular lumen.

2. A vascular catheter as in claim 1, wherein the macroporous matrix is disposed at or near the distal end of the catheter body.

3. A vascular catheter as in claim 2, wherein the macroporous matrix extends over a length in the range from about 2 cm to 50 cm.

4. A catheter as in claim 1, wherein the macroporous matrix has a molecular weight cutoff above about 10,000 daltons.

5. A catheter as in claim 1, wherein the macroporous matrix comprises a woven fabric.

6. A catheter as in claim 1, wherein the macroporous matrix comprises a non-woven fabric.

7. A catheter us in claim 1, wherein the macroporous matrix comprises a fabric having fibers composed of a material selected from the group consisting of polyethylene, polypropylene, polyester, nylon, polytetrafluoroethylene, polycarbonate, polystyrene, cellulose, and polyacetonitrile.

8. A catheter as in claim 7, wherein the macroporous matrix comprises a woven polyethylene terephthalate fabric.

9. A catheter as in claim 1, wherein the macroporous matrix comprises an organic polymer membrane.

10. A catheter as in claim 1, further comprising means for partitioning the annular lumen into at least two isolated regions and means for separately delivering fluid to each region.

11. A catheter as in claim 10, wherein the means for partitioning comprises an annular barrier extending between the inner flexible tubular member and the outer flexible tubular member.

12. A catheter as in claim 11, wherein the means for separately delivering fluids comprises at least two isolated lumens extending from the proximal end of the inner tubular member to the region.

13. A vascular perfusion catheter comprising: an inner flexible tubular member having a proximal end, a distal end, and a central lumen extending between the proximal and distal ends;
an outer flexible tubular member disposed coaxially about the inner flexible tubular member and sealed thereto near the distal end to define an annular lumen having a closed distal end, wherein at least a portion of the outer flexible tubular member near the distal end is formed from a macroporous matrix material which will allow the controlled diffusion of macromolecules from the annular lumen; and
at least one Doppler crystal attached to the catheter for measuring blood flow past the catheter.

FIG. 1

EP 0 511 499 A2

FIG. 2

FIG. 3

FIG. 4

FIG. 5

FIG. 6

FIG. 7          FIG. 8          FIG. 9

FIG. 10

FIG. 11  FIG. 12  FIG. 13

300

*FIG. 14*

*FIG. 15*  *FIG. 16*  *FIG. 17*

FIG. 18